# EUROPÄISCHE PATENTSCHRIFT

(11) **EP 0 436 075 B1**
(45) Veröffentlichungstag und Bekanntmachung des Hinweises auf die Patenterteilung: **07.02.1996**
(21) Anmeldenummer: 90118917.5
(22) Anmeldetag: 04.10.1990
(51) Int. Cl.: A61L 15/28

(54) **Hygienische Absorptionsvorlage**
Absorbent sanitary article
Garniture hygiénique absorbante

(30) Priorität: 30.12.1989 DE 3943404
(43) Veröffentlichungstag der Anmeldung: 10.07.1991
(73) Patentinhaber: VP - SCHICKEDANZ AG, D-90022 Nürnberg (DE)
(72) Erfinder: Reinheimer, Horst, Dr., W-8501 Heroldsberg (DE); Rink, Norbert, Dr., W-8508 Wendelstein 2 (DE)
(74) Vertreter: Hübner, Gerd, Dipl.-Phys.

(56) Entgegenhaltungen:
- FR-A- 1 530 716

## Beschreibung

Die Erfindung betrifft eine hygienische Absorptionsvorlage, beispielsweise eine Kinder- oder Erwachsenenwindel, eine Damenbinde, Slipeinlage, eine Krankenunterlage oder dergl. Derartige Vorlagen weisen sämtlich wenigstens einen Saugkörper auf, der im wesentlichen aus Zellstoff-Flocken besteht. Zur Erhöhung der Saugleistung können auch noch synthetische und natürliche Quellstoffe zugesetzt werden, welche heute handelsüblich sind und beispielsweise aus hydrophilen Polyacrylaten, Polystyrolsulfonaten oder ähnlichen Stoffen bestehen. Der Saugkörper kann gegebenenfalls mit einer flüssigkeitsdurchlässigen Folie, beispielsweise einer Vliesstoffbahn oder auch einer durchlöcherten Polyethylenfolie umhüllt oder abgedeckt sein. Des weiteren ist es bekannt, an der Unterseite des Saugkörpers eine flüssigkeitsdichte Sperrschicht anzubringen, welche meist aus einer dünnen Polyethylenfolie, mitunter aber auch aus anderen Kunststoffschichten besteht.

Hygienische Absorptionsvorlagen dieser Art sind bekannt und werden in großem Umfange verwendet. Zu ihrer Herstellung wird meist gebleichter Sulfit- oder Sulfat-Zellstoff verwendet, der durch Trockenzerfasern entsprechender Zellstofftafeln oder -bahnen aufbereitet wird. Der auf diese weise gewonnene Flockenzellstoff (Fluff) wird von einem Luftstrom auf eine Siebunterlage geblasen und dort entsprechend den Abmessungen der jeweils herzustellenden Absorptionsvorlage abgelegt. Nach der fertigen Ausbildung des Saugkörpers wird dieser in der schon angedeuteten Weise durch Umhüllung, gegebenenfalls aber auch Verklebung, Verdichtung usw. weiterverarbeitet.

Aus der FR-A-1 530 716 ist es bekannt, einen Saugkörper einer hygienischen Absorptionsvorlage aus ungebleichtem Sulfat- Zellstoff herzustellen, um einen höhere Formstabilität des Saugköpers zu erzielen.

Es ist ebenfalls bekannt, dem Zellstoff als preiswertes Streckungsmittel Holzschliff, beispielsweise aus Kiefernholz zuzusetzen, welcher zuvor zu Platten verpreßt worden ist, worauf die Platten in gleicher Weise wie der Fluff-Zellstoff mechanisch in Hammermühlen zerkleinert und gemahlen werden (DE-AS 1 259 014). Es ist bekannt, daß man auf diese Weise hochsaugfähige Saugkörper erhält, deren mechanische Festigkeit allerdings gering ist. Das gleiche gilt auch für den heute mitunter eingesetzten Rohstoff CTMP (Chemo-Thermo-Mechanical Pulp)

Der Erfindung liegt die Aufgabe zugrunde, die vorbekannten hygienischen Absorptionsvorlagen dahingehend weiterzuentwickeln, daß sie bei erhöhter Formstabilität ein zumindest gleiches, vorzugsweise aber ebenfalls erhöhtes Flüssigkeitsaufsaugvermögen haben. Außerdem wird angestrebt, daß die Vorlagen in rohstoff- und umweltschonender Weise mit geringen Kosten hergestellt werden können.

Zur Lösung dieser Aufgabe wird vorgeschlagen, daß die Zellstoff-Flocken wenigstens zum Teil aus ungebleichtem Sulfat-Zellstoff bestehen, der einen Rest-Lignin-Gehalt von 2-6 Gewichtsprozent aufweist. Bei Vergleichsversuchen wurde festgestellt, daß ein solcher ungebleichter Sulfat-Zellstoff überraschenderweise eine gegenüber gebleichtem Zellstoff erhöhte Formstabilität aufweist, wenn aus diesem Zellstoff Saugkörper für hygienische Absorptionsvorlagen geformt worden sind. Dieses Ergebnis ist um so überraschender als festgestellt wurde, daß ungebleichter Sulfit-Zellstoff diese Eigenschaften nicht aufweist, sondern im Gegenteil eine besonders geringe Formstabilität hat, die etwa mit der Formstabilität von Holzschliff vergleichbar ist, wie dies in der DE-AS 1 259 014 beschrieben ist. Hinzu kommt, daß der Rohstoff CTMP Flockenkörper mit ebenfalls sehr geringer Formstabilität ergibt.

Bei vergleichenden Untersuchungen verschiedener Zellstoffsorten hat sich herausgestellt, daß zwei Einflußgrößen für die erstrebte hohe Formstabilität verantwortlich sind, nämlich einerseits der Zellstofftyp und andererseits der Lignin-Gehalt. Als optimaler Zellstofftyp hat sich Sulfat-Zellstoff erwiesen, wenn eben den erwähnten Rest-Lignin-Gehalt von 2 bis 6 Gewichtsprozent aufweist.

In der Zellstoff-Technologie ist es üblich, als Maß für den Lignin-Gehalt den sog. Kappawert anzugeben. Dieser Wert wird ermittelt, indem eine definierte Einwaage des zu untersuchenden Zellstoffes in Wasser von Raumtemperatur suspendiert, mit Schwefelsäure angesäuert und mit einer ebenfalls definierten Menge von 0,1 normaler KMnO₄-Lösung versetzt wird. Die Probe wird dabei eine bestimmte Zeit von meist 10 Minuten gerührt, worauf der noch verbleibende Überschuß an Kaliumpermanganat-Lösung jodometrisch bestimmt wird. Eine genaue Beschreibung des Verfahrens findet man in dem Buch "Zellstoff - Papier", VEB-Verlag Leipzig, 5. Auflage 1978, Seite 178.

Typisch und kennzeichnend für vorzugsweise zu verwendende Sulfat-Zellstoff-Flockensorten ist ein Kappawert von 10 bis 40. Als optimal hat es sich auch erwiesen, wenn die Zellstoff-Flocken aus solchem ungebleichtem Sulfat-Zellstoff durch mechanisches Zerfasern hergestellt werden, der vor der Zerfaserung eine Dichte von ≦ 650 kg/m³ aufgewiesen hat.

Typisch für die erfindungsgemäß aufgebauten hygienischen Absorptionsvorlagen ist neben ihrer hohen Saugkapazität ihre beachtliche Formstabilität. Diese Formstabilität kann qualitativ leicht beobachtet werden, wenn man auf einen freigelegten, also von der Folie befreiten Fluffsaugkörper einen Luftstrom richtet. Wenig formstabile Saugkörper, beispielsweise solche mit einem nennenswerten Gehalt an Holzschliff, werden dabei in wenigen Sekunden voll ständig abgetragen. Zur quantitativen Bestimmung der Formstabilität kann so vorgegangen werden, daß eine genormte Probe (beispielsweise 10 x 10 cm²) des Saugkörpers gewogen und auf ein Sieb aufgelegt wird. Das Sieb befindet sich in einem geschlossenen etwa halbkugelförmigen Raum, in welchen von unten durch das Sieb in definierter Weise ein ebenfalls definierter Luftstrom eingeblasen wird. Bei wenig formstabilen Saugkörpern werden die Flocken durch diesen Luftstrom in kurzer Zeit auseinandergeblasen und sammeln sich im halbkugelförmigen Raum an. Nach Durchführung einer definierten Belastung dieser Art wird der Luftstrom unterbrochen und der noch auf dem Sieb verbliebene Flockenkörper zurückgewogen. Bildet man den Quotienten aus Auswaage und Einwaage und multipliziert diesen mit 100, so erhält man ein Maß für die Formstabilität in Prozent.

Die für die Gebrauchseigenschaften von hygienischen Absorptionsvorlagen ebenfalls wichtige Eigenschaft des Flüssigkeitsaufnahmevermögens wird üblicherweise dadurch gemessen, daß eine definierte und gewogene Probe des Saugkörpers unter praxisgleicher Gewichtsbelastung solange mit Harnersatzflüssigkeit beaufschlagt wird, bis die Probe gesättigt ist. Danach wird die Probe Zurückgewogen und die Gewichtsaufnahme bestimmt.

Die nachfolgende Tabelle zeigt die Leistungsdaten verschiedener Saugkörper für hygienische Absorptionsvorlagen, die unter Verwendung unterschiedlicher Zell stoffsorten hergestellt worden sind.

| | gebleichter Zellstoff | CTMP | ungebleichter Sulfit-Zellstoff | ungebleichter Sulfat-Zellstoff |
|---|---|---|---|---|
| Lignin-Gehalt (%) | ≦ 1 | 24 - 27 | 1,5 - 6 | 2 - 6 |
| Flockenkissenstabilität % (bei 0,1 kg/m³ Dichte) | 75 | 0 - 25 | 0 - 10 | 60 - 90 |
| Harnersatzaufnahme x-fach in g/g unter Belastung (50 g/cm²) | ca. 6,5-7,7 | 8,0 ± 0,2 | ca. 8,1 ± 0,2 | bis zu 8,6 |

Es ist erkennbar, daß gebleichter Zellstoff eine verhältnismäßig hohe Flockenkissenstabilität bei mittlerer Flüssigkeitsaufnahme zeigt. Ungebleichter Sulfit-Zellstoff zeigt demgegenüber zwar eine erhöhte Flüssigkeitsaufnahme, jedoch eine rapid abgesunkene Flockenkissenstabilität. Das gleiche gilt auch für den Rohstoff CTMP. Von diesen Werten heben sich die entsprechenden Werte für ungebleichten Sulfat-Zellstoff vorteilhaft ab. Beide Werte für die Flockenkissenstabilität und für das Flüssigkeitsaufnahmevermögen sind gegenüber gebleichten Zellstoff-Sorten angehoben.

Die Tabelle zeigt ferner den Einfluß des Lignin-Gehaltes. Gebleichter Zellstoff mit Lignin-Gehalten unter 1 % hat die gewohnten mittleren Eigenschaften. CTMP-Sorten mit sehr hohem Lignin-Gehalt zeigen ebenfalls geringe Flockenkissenstabilität. Erst wenn die beiden Erfordernisse "Sulfat-Zellstoff" einerseits und "ungebleichte Qualität" andererseits mit mittleren Lignin-Gehalten von 2 bis 6 % zusammenkommen, ergeben sich die erstrebten verbesserten Werte.

## Patentansprüche

1. Hygienische Absorptionsvorlage mit einem Saugkörper, der im wesentlichen aus Zellstoff-Flocken besteht und gegebenenfalls mit einer flüssigkeitsdurchlässigen Folie umhüllt oder abgedeckt ist, wobei die Zellstoff-Flocken wenigstens zum Teil aus ungebleichtem Sulfat-Zellstoff bestehen,
dadurch gekennzeichnet,
daß die Zellstoff-Flocken einen Rest-Lignin-Gehalt von 2 - 6 Gewichtsprozent aufweisen.

2. Hygienische Absorptionsvorlage nach Anspruch 1, dadurch gekennzeichnet,
daß die Zellstoff-Flocken einen Kappawert von 10 - 40 aufweisen.

3. Hygienische Absorptionsvorlage nach Anspruch 1 oder 2, dadurch gekennzeichnet,
daß die Zellstoff-Flocken aus ungebleichtem Sulfat-Zellstoff durch mechanisches Zerfasern hergestellt worden sind, der vor der Zerfaserung eine Dichte von ≦ 650 kg/m³ aufgewiesen hat.

## Claims

1. Absorbent sanitary article comprising an absorbent body, which consists substantially of cellulose flakes and, if required, is enclosed or covered by a liquid-permeable film, the cellulose flakes consisting at least in part of unbleached sulfate cellulose, characterized in that the cellulose flakes have a lignin residue of 2 - 6 percent by weight.

2. Absorbent sanitary article according to claim 1, characterized in that the cellulose flakes have a kappa number of 10 - 40.

3. Absorbent sanitary article according to claim 1 or 2, characterized in that the cellulose flakes have been produced by mechanical defibration from unbleached sulfate cellulose of a mass density of ≦ 650 kg/m³ prior to the defibration.

## Revendications

1. Garniture hygiénique absorbante comprenant un corps absorbant, qui est essentiellement constitué de flocons de cellulose et, le cas échéant, est enveloppé ou recouvert d'une feuille perméable aux liquides, les flocons de cellulose étant constitués, au moins en partie, de cellulose au sulfate non blanchie ou écrue, caractérisée en ce que les flocons de cellulose présentent une teneur résiduelle de lignine de 2 à 6 pour-cent en poids.

2. Garniture hygiénique absorbante selon la revendication 1, caractérisée en ce que les flocons de cellulose présentent un indice kappa de 10 à 40.

3. Garniture hygiénique absorbante selon la revendication 1 ou 2, caractérisée en ce que les flocons de cellulose ont été fabriqués par défibrage mécanique, en cellulose au sulfate, non blanchie, qui avant le défibrage présentait une densité ≦ 650 kg/m³.
